# EUROPEAN PATENT APPLICATION

(11) **EP 0 896 824 A1**
(43) Date of publication of application: **17.02.1999**
(21) Application number: 97113466.3
(22) Date of filing: 05.08.1997
(51) Int. Cl.: A61K 35/16

(54) **A universally applicable blood plasma**

(71) Applicant: OCTAPHARMA AG, 8866 Ziegelbrücke (CH)
(72) Inventor: Marguerre, Wolfgang, S-25284 Helsingborg (SE); Svae, Tor-Einar, N-2090 Hurdal (NO)
(74) Representative: Meyers, Hans-Wilhelm, Dr.Dipl.-Chem.

(57) **Abstract**

A universally applicable blood plasma obtainable by a process comprising the steps of mixing blood or blood plasma of blood groups A and B optionally blood or blood plasma of blood group AB without admixing substantial amounts of blood or blood plasma derived from blood group 0.

## Description

The present invention is related to a universally applicable blood plasma as well as a process for preparing same.

Blood plasma is a very widely used substitute for blood losses, for example, during operation or when severe bleedings occur after accidents. Since there are four major blood groups, at present different plasmas are prepared to serve patients with the different blood groups. Obviously, this is awkward since four different blood plasma preparations have to be stored by the respective blood banks or blood centers in the hospital. Furthermore, it would be necessary to determine the blood group of the patient who is in need of a blood substitute. This delay might be critical in case of emergency.

Thus, it is one object of the invention to provide a blood plasma preparation which can be applied universally to patients with different blood groups.

The present invention provides a universally applicable blood plasma obtainable by mixing blood or blood plasma of blood groups A and B and optionally blood or blood plasma derived from blood group AB, without admixing substantial amounts of blood or blood plasma derived from blood group 0.

The blood plasma preparation of the invention is advantageous since there will be no risk of incompatible plasma infusions which may cause severe adverse reactions which can even be lethal. Additionally, the blood plasma preparation of the invention can be located at the site of the intended use, i. e. operation rooms and emergency rooms. Hence, the end user can have access to this lifesaving product immediately on request. The respective end user does not have to wait until the product ordered from the blood centers is released. At present the blood centers have to release a blood group specific plasma according to the blood group of the recipient.

In a preferred embodiment of the present invention the AB0 blood group specific antibodies of the blood plasma are neutralized and/or removed.

A blood plasma preparation which is substantially free of fractions of group 0 leads to a more universally applicable blood plasma preparation. The blood plasma of the invention comprises preferably high amounts of blood plasma derived from donors having the blood group A, medium amounts of blood plasma derived from donors having the blood group B and optionally low amounts of blood plasma derived from donors having the blood group AB. The blood plasma of the invention is substantially free of blood plasma from donors having the blood group 0. A further preferred embodiment of the present invention is a blood plasma comprising 6 to 10 parts of blood plasma derived from donors having the blood group A, 1 to 3 parts of blood plasma derived from donors having the blood group B, and 0.0 to 1.5 parts of blood plasma derived from donors having the blood group AB and substantially no blood plasma derived from blood group 0.

In a very preferred embodiment of the present invention the blood plasma comprises 7.5 to 8.5 parts of blood plasma derived from donors having the blood group A, 1.5 to 2.5 parts of blood plasma derived from donors having the blood group B, and optionally about 1 part of blood plasma derived from donors having the blood group AB and substantially no blood or blood plasma derived from blood group 0.

Preferably, the blood plasma of the invention has been prepared by pooled plasma derived from any donors. The pooled plasma preferably has been virus inactivated. The virus inactivation can be performed prior to mixing blood or blood plasma of different blood groups A, B and AB or after preparing of the blood plasma of the present invention. For virus inactivation any methods of the art can be used, for example, virus inactivation by irradiation with actinic radiation, pasteurization, solvent detergent treatment or combinations of the method. A well known method in the art, for example, is the solvent detergent treatment as disclosed in EP-A-0 131 740 as well as the method according to WO-A-94/17834 developed by Octapharma AG, Switzerland.

The blood plasma of the invention can be stored and delivered in any state known to the skilled person. The blood plasma may contain pharmaceutically acceptable adjuvants, such as stabilizers and anticoagulants.

Preferably, the blood plasma of the invention is stored or delivered in a solid state, for example, in frozen form. Furthermore, it may be advantageous to store or deliver the blood plasma of the invention in a lyophilized or spray-dried form. In case the dried plasma is needed it can easily be dissolved in sterile water in order to infuse it in the patient.

The AB0 blood group specific antibody titre of the blood plasma of the invention is preferably lower than 16 for anti A/anti B IgM and 64 for anti A/anti B IgG. In a very preferred embodiment the titre of the anti-A and anti-B antibodies is lower than 8 for IgM and lower than 32 for IgG.

The process for preparing the blood plasma of the invention comprises the steps of pooling blood or blood plasma of donors having the blood groups A, B and optionally AB as well as neutralizing and/or removing antibodies.

If blood is used as a starting material, blood plasma is produced from the blood pool by methods known in the art.

More than two-thirds of all blood donors have free A and/or B substance in plasma. These substances are almost identical to A and B antigenes bound to the surface of red blood cells. By mixing appropriate amounts of blood or blood plasma of the blood groups A, B and optionally AB anti-A and anti-B antibodies of subclasses IgM and IgG are neutralized by binding two free A and/or B substances and/or are removed during the further processing.

Surprisingly, although the plasma of the present invention used as raw material contains both residual red blood cells and the complete complement systems there are no signs of complement activation during the production or in the final product. According to the manufacturing process it is preferred that the mixing takes place during pooling of the plasma units in the beginning of the process combined with a complete cell removal and a virus inactivation process, preferably a solvent detergent treatment. The final product can be used without limitation on the infusion rate and total dosage.

The blood plasma of the invention prepared according to the process of the invention is advantageous since it additionally is coagulation active.

The present invention is further illustrated but not limited by the following example.

### Example

278 l of fresh-frozen plasma derived from blood group A, 68 l of B and 34 l of AB are mixed together and allowed to thaw. Sodium dihydrogenphosphate dihydrate is added as a buffer to stabilize the plasma proteins. After filtration through a membrane having a pore size of 1 µm, the obtained fraction is virus inactivated by the solvent detergent method. After removal of the virus inactivating agents, glycine is added to adjust the osmolarity. During qualitiy control the amount of free anti-A and anti-B antibodies is tested. Such tests are well-known in the art. The titre of anti-A and anti-B antibodies should be < 8 for IgM and < 32 for IgG.

## Claims

1. A universally applicable blood plasma obtainable by a process comprising the steps of mixing blood or blood plasma of blood groups A and B optionally blood or blood plasma of blood group AB without admixing substantial amounts of blood or blood plasma derived from blood group 0.

2. The blood plasma of claim 1, wherein the AB0 blood group specific antibodies of the blood plasma are neutralized and/or removed.

3. The blood plasma of claim 2, comprising high amounts of blood plasma derived from donors having the blood group A, medium amounts of blood plasma derived from donors having the blood group B, and optionally low amounts of blood plasma derived from donors having the blood group AB.

4. The blood plasma of claim 3, comprising
- 6 to 10 parts of blood or blood plasma derived from donors having the blood group A,
- 1 to 3 parts of blood or blood plasma derived from donors having the blood group B,
- 0.0 to 1.5 parts of blood or blood plasma derived from donors having the blood group AB,
- substantially no blood or blood plasma derived from donors having the blood group 0.

5. The blood plasma of any one of the foregoing claims in liquid, frozen, or dried state.

6. The blood plasma of claim 5 in lyophilized or spray-dried form.

7. The blood or blood plasma of any one of the foregoing claims having pharmaceutically acceptable adjuvants, such as stabilizers and anticoagulants.

8. The blood or blood plasma of any one of the foregoing claims which blood plasma was subjected to a treatment for virus inactivation.

9. A process of preparing the blood or blood plasma of claim 1 comprising the steps of
- pooling blood or blood plasma of donors having the blood groups A, and B,
- optionally admixing blood or blood plasma of donors having the blood group AB,
- optionally producing plasma from the blood or blood plasma pool,
- neutralizing and/or removing AB0 blood group specific antibodies.

10. The process of claim 9 further comprising the step of spray-drying or lyophilizing the plasma.
